# EUROPÄISCHE PATENTSCHRIFT

(11) **EP 3 559 465 B1**
(45) Veröffentlichungstag und Bekanntmachung des Hinweises auf die Patenterteilung: **09.12.2020**
(21) Anmeldenummer: 17816854.8
(22) Anmeldetag: 18.12.2017
(51) Int. Cl.: F04B 43/073

(54) **BETÄTIGUNGSEINRICHTUNG UND VERFAHREN ZUM BETREIBEN EINER BETÄTIGUNGSEINRICHTUNG SOWIE MEMBRANPUMPE MIT EINER BETÄTIGUNGSEINRICHTUNG UND EINER MEMBRANPUMPENEINRICHTUNG UND EINE BLUTBEHANDLUNGSVORRICHTUNG MIT EINER MEMBRANPUMPE**
OPERATING DEVICE, METHOD FOR OPERATING AN OPERATING DEVICE, DIAPHRAGM PUMP HAVING AN OPERATING DEVICE AND A DIAPHRAGM PUMP DEVICE, AND A BLOOD TREATMENT APPARATUS HAVING A DIAPHRAGM PUMP
DISPOSITIF D'ACTIONNEMENT ET PROCÉDÉ POUR FAIRE FONCTIONNER UN DISPOSITIF D'ACTIONNEMENT AINSI QUE POMPE À MEMBRANE POURVUE D'UN DISPOSITIF D'ACTIONNEMENT ET D'UN DISPOSITIF DE POMPE À MEMBRANE, ET SYSTÈME DE TRAITEMENT DU SANG POURVU D'UNE POMPE À MEMBRANE

(30) Priorität: 21.12.2016 DE 102016015207
(43) Veröffentlichungstag der Anmeldung: 30.10.2019
(73) Patentinhaber: Fresenius Medical Care Deutschland GmbH, 61352 Bad Homburg v.d.H (DE)
(72) Erfinder: ANDERLE, Jens, 73326 Deggingen (DE); WIRTL, Hannes, 86956 Schongau (DE)
(74) Vertreter: Oppermann, Frank
(86) Internationale Anmeldenummer: PCT/EP2017/083404
(87) Internationale Veröffentlichungsnummer: WO 2018/114862

(56) Entgegenhaltungen:
- EP-A2- 1 353 069
- DE-A1-102013 102 397
- US-A1- 2008 202 591

## Beschreibung

Die Erfindung betrifft eine Betätigungseinrichtung zur mechanischen Ansteuerung einer Membranpumpeneinrichtung und ein Verfahren zum Betreiben einer derartigen Betätigungseinrichtung. Darüber hinaus betrifft die Erfindung eine Membranpumpe, die über eine derartige Betätigungseinrichtung und eine derartige Membranpumpeneinrichtung verfügt, sowie eine Blutbehandlungsvorrichtung, insbesondere Dialysevorrichtung, die eine derartige Membranpumpe aufweist.

Zur Förderung von medizinischen Flüssigkeiten finden in der Medizintechnik Membranpumpen Verwendung. Der Vorteil von Membranpumpen liegt in der Trennung der Antriebseinheit von dem zu fördernden Fluid durch eine Membran.

In der Medizintechnik werden an Membranpumpen hohe Anforderungen gestellt. Für bestimmte Anwendungen sollen die Membranpumpen auch bei niedrigen Flüssen eine hohe Fördergenauigkeit haben. Eine besonders genaue Dosierung bei einer niedrigen Flussrate erfordert beispielsweise die Citratantikoagulation (CiCa-Antikoagulation) in der Dialyse.

Es sind Membranpumpen bekannt, die über eine nur zur einmaligen Verwendung bestimmte Membranpumpeneinrichtung verfügen. Die Membranpumpeneinrichtung dieser Membranpumpen kann als eine Einwegkassette ausgebildet sein, in der eine Ausnehmung ausgebildet ist, die von einer elastischen Membran verschlossen ist. Während der Saugphase wird die Fluidströmung aus der Pumpenkammer unterbrochen und während der Druckphase wird die Fluidströmung in die Pumpenkammer unterbrochen. Der Antrieb der elastischen Membran kann mit einer Betätigungseinrichtung erfolgen, die nicht zur einmaligen Verwendung bestimmt ist.

Eine Betätigungseinrichtung wird dazu eingesetzt, eine Membranpumpeneinrichtung mechanisch anzusteuern, um ein Fluid, bei dem es sich insbesondere um eine Flüssigkeit handeln kann, von einem Eingangsanschluss der Membranpumpeneinrichtung zu einem Ausgangsanschluss der Membranpumpeneinrichtung zu fördern. Beispielhaft umfasst die Membranpumpeneinrichtung einen plattenförmig ausgebildeten Kanalkör- per, an dem ausgehend von einem Eingangsanschluss ein Saugkanal bis zu einer Pumpkammer erstreckt ist und an dem ausgehend von der Pumpkammer ein Druckkanal bis zu einem Ausgangsanschluss erstreckt ist. Dabei ist vorgesehen, dass die Pumpkammer als Ausnehmung in eine Oberfläche des Kanalkörpers eingebracht ist und von einer abdichtend am Kanalkörper angebrachten Membran verschlossen ist. Durch eine Bewegung der Membran wird ein Volumen der Pumpkammer verändert, wodurch unter der zusätzlichen Voraussetzung einer zyklischen Blockierung und Freigabe des Saugkanals und des Druckkanals ein Fluidmassenstrom für das Fluid hervorgerufen werden kann.

Aus der DE 1 353 069 A2 ist eine gattungsgemäße Betätigungseinrichtung zur Ansteuerung einer Membranpumpeneinrichtung zur Förderung von Fluiden bekannt, die einen Grundkörper aufweist, der eine Montagefläche für die Membranpumpeneinrichtung umfasst und der von einer Steuerfluidleitung durchsetzt ist, die sich von einem Steuerfluidanschluss bis zu einer Steuerfluidmündung erstreckt, die an der Montagefläche ausmündet. Darüber hinaus weist die Betätigungseinrichtung ein Arbeitsfluidventil auf, das für eine Beeinflussung eines Querschnitts einer Arbeitsfluidleitung ausgebildet ist, die den Grundkörper durchsetzt. Das Steuerfluidventil und das Arbeitsfluidventil sind elektrisch mit einer Steuereinrichtung verbunden, die für eine elektrische Ansteuerung des Steuerfluidventils und des Arbeitsfluidventils ausgebildet ist.

Die DE 10 2013 102 397 A1 beschreibt ein Dosiersystem zum Dosieren eines Fluids mit einer Dosiereinheit, einer Einrichtung zur Versorgung der Dosiereinheit mit dem zu dosierenden Fluid und einem Steuermodul, das ein Steuerfluid zur Dosiereinheit leitet. Die Dosiereinheit weist ein erstes Gehäuseteil, ein zweites Gehäuseteil und eine zwischen den Gehäuseteilen angeordnete Membran auf, die einen zwischen den Gehäuseteilen gebildeten Hohlraum in eine Pumpkammer und eine Dosierkammer teilt. In dem ersten Gehäuseteil sind ein Steuerfluid aufnehmende Kanäle ausgebildet sind, die mit dem Steuermodul in Strömungsverbindung stehen, und in dem zweiten Gehäuseteil sind ein Dosierfluid aufnehmende Kanäle ausgebildet sind, die mit der Einrichtung zur Versorgung der Dosiereinheit mit dem zu dosierenden Fluid in Strömungsverbindung stehen.

Die US 2008/0202591 A1 beschreibt ein Kassettensystem, das eine Kassette zum Mischen, eine Kassette zum Bilanzieren und eine mittlere Kassette umfasst, wobei die Kassette zum Mischen mittels einer Fluidleitung mit der mittleren Kassette und die mittlere Kassette mittels einer Fluidleitung mit der Kassette zum Bilanzieren verbunden ist.

Die Aufgabe der Erfindung liegt darin, eine Betätigungseinrichtung zur Ansteuerung einer Membranpumpeneinrichtung zu schaffen, die eine zuverlässige Durchführung eines Fördervorgangs einer Membranpumpeneinrichtung ermöglicht, und ein Verfahren zum Betreiben einer Betätigungseinrichtung bereitzustellen, das eine zuverlässige Durchführung eines Fördervorgangs einer Membranpumpeneinrichtung erlaubt.

Eine weitere Aufgabe der Erfindung ist, die Fördergenauigkeit einer Membranpumpe zur Förderung einer medizinischen Flüssigkeit zu verbessern, insbesondere, eine Membranpumpe zu schaffen, die eine besonders genaue Dosierung von Fluiden, beispielsweise einer Lösung zur CiCa-Antikoagulation, auch bei sehr kleinen Flussraten erlaubt, wobei ein konstanter Fluidmassenstrom gewährleistet werden soll.

Darüber hinaus liegt der Erfindung die Aufgabe zugrunde, eine Blutbehandlungsvorrichtung, insbesondere eine Dialysevorrichtung, mit einer Membranpumpe bereitzustellen, die eine besonders genaue Dosierung eines Fluids, beispielsweise einer Lösung zur CiCa-Antikoagulation, auch bei sehr kleinen Flussraten mit einem konstanten Fluidmassenstrom erlaubt.

Die Lösung dieser Aufgaben erfolgt erfindungsgemäß mit den Merkmalen der unabhängigen Patentansprüche. Die abhängigen Ansprüche betreffen vorteilhafte Ausführungsformen der Erfindung.

Die erfindungsgemäße Betätigungseinrichtung weist einen Grundkörper auf, der eine Montagefläche für eine Membranpumpeneinrichtung umfasst und der von einer Steuerfluidleitung durchsetzt ist, die sich von einem Steuerfluidanschluss bis zu einer Steuerfluidmündung erstreckt, die an der Montagefläche ausmündet, wobei in der Steuerfluidleitung ein Steuerfluidventil angeordnet ist, das für eine Beeinflussung eines Querschnitts der Steuerfluidleitung ausgebildet ist. Ferner umfasst die Betätigungseinrichtung eine Stelleinrichtung, die einen an der Montagefläche angeordneten Fluidaktor zur Bereitstellung einer Stellbewegung und ein Arbeitsfluidventil aufweist, das für eine Beeinflussung eines Querschnitts einer Arbeitsfluidleitung ausgebildet ist, die den Grundkörper durchsetzt, und wobei das Steuerfluidventil und das Arbeitsfluidventil elektrisch mit einer Steuereinrichtung verbunden sind, die für eine elektrische Ansteuerung des Steuerfluidventils und des Arbeitsfluidventils ausgebildet ist.

Der Grundkörper kann wahlweise einstückig oder mehrteilig ausgebildet sein und ist zumindest mit einer als Montagefläche bezeichneten Schnittstelle für die Ankopplung einer Membranpumpeneinrichtung ausgestattet. Die Montagefläche kann in Abhängigkeit von der Ausgestaltung der Membranpumpeneinrichtung als Ebene oder als dreidimensional strukturierte Fläche, insbesondere mit ebenen Bereichen, ausgebildet sein. Um eine Betätigung der, insbesondere als vollständig passives Bauelement ohne eigene Aktorik und/oder Sensorik ausgebildeten, Membranpumpeneinrichtung zu ermöglichen, ist der Grundkörper von einer Steuerfluidleitung durchsetzt, die sich von einem Steuerfluidanschluss bis zu einer Steuerfluidmündung erstreckt. Der Steuerfluidanschluss kann unmittelbar am Grundkörper angeordnet sein oder beabstandet vom Grundkörper ausgebildet sein, wobei dann beispielsweise eine Schlauchverbindung zwischen dem Steuerfluidanschluss und der Steuerfluidleitung vorgesehen sein kann. Die Steuerfluidleitung erstreckt sich ausgehend vom Steuerfluidanschluss bis zur Steuerfluidmündung, die an der Montagefläche ausmündet. An dem Steuerfluidanschluss kann beispielsweise eine Steuerfluid- quelle, insbesondere eine Druckluftquelle, oder eine Steuerfluidsenke, insbesondere ein Vakuumerzeuger, angeschlossen werden. Mit Hilfe des Steuerfluids wird eine Bewegung einer Pumpmembran der Membranpumpeneinrichtung durch zyklische oder azyklische Druckbeaufschlagung und/oder Unterdruckbeaufschlagung hervorgerufen, wodurch die Pumpmembran ein zu förderndes Fluid in eine Pumpkammer ansaugen und/oder aus der Pumpkammer ausstoßen/austragen kann. Um Einfluss auf einen Steuerfluidstrom nehmen zu können, ist in der Steuerfluidleitung ein Steuerfluidventil angeordnet, das für eine Beeinflussung eines Querschnitts der Steuerfluidleitung, insbesondere zwischen einer Schließstellung für eine vollständige Blockierung der Steuerfluidleitung und einer Öffnungsstellung für eine vollständige Freigabe des Querschnitts der Steuerfluidleitung, ausgebildet ist.

Dem Grundkörper ist weiterhin eine Stelleinrichtung zugeordnet, die für eine mechanische Betätigung eines an der Membranpumpeneinrichtung vorgesehenen Membranventils ausgebildet ist. Dieses Membranventil kann beispielsweise fluidisch kommunizierend mit der ebenfalls in der Membranpumpeneinrichtung ausgebildeten Pumpkammer verbunden sein und für eine Steuerung eines Fluidstroms in die Pumpkammer oder aus der Pumpkammer eingerichtet sein. Zur Betätigung dieses Membranventils weist die Stelleinrichtung einen an der Montagefläche angeordneten Fluidaktor auf, bei dem es sich beispielhaft um einen kompakt bauenden Pneumatikzylinder handelt. Die Stelleinrichtung umfasst ein bewegliches Aktorglied, das linear und/oder rotatorisch zwischen einer Ruhestellung und einer Funktionsstellung bewegt werden kann und insbesondere in der Funktionsstellung über die Montagefläche abragt. Hierdurch kann eine Membran des gegenüberliegend anbringbaren Membranventils der Membranpumpeneinrichtung lokal deformiert werden, um beispielsweise eine Schließstellung dieses Membranventils herbeizuführen. Ferner kann vorgesehen sein, dass das Membranventil sich in einer Öffnungsstellung befindet, wenn das Aktorglied die, insbesondere flächenbündig mit der Montagefläche ausgebildete, Ruhestellung einnimmt. Für eine fluidische Ansteuerung des Fluidaktors der Stelleinrichtung ist ein Arbeitsfluidventil vorgesehen, das einer Arbeitsfluidleitung zugeordnet ist, die den Grundkörper durchsetzt. Dabei ist das Arbeitsfluidventil beispielhaft zwischen einer Schließstellung für eine vollständige Blockierung der Arbeitsfluidleitung und einer Öffnungsstellung für eine vollständige Freigabe des Querschnitts der Arbeitsfluidleitung umschaltbar oder einstellbar. Eine Bereitstellung des Arbeitsfluids für die Betätigung des Fluidaktors kann beispielhaft über einen separat ausgebildeten Arbeitsfluidanschluss erfolgen, an dem eine Fluidquelle oder eine Fluidsenke für eine Bereitstellung eines druckbeaufschlagten Arbeitsfluids oder eines Unterdrucks angeschlossen werden können. Alternativ kann eine Verbindung der Arbeitsfluidleitung mit dem Steuerfluidanschluss vorgesehen sein.

Sowohl das Steuerfluidventil als auch das Arbeitsfluidventil sind elektrisch mit einer Steuereinrichtung verbunden, die für eine elektrische Ansteuerung des Steuerfluidventils und des Arbeitsfluidventils ausgebildet ist. Beispielhaft kann es sich bei der Steuereinrichtung um einen Mikroprozessor oder Mikrocontroller handeln, die zur Abarbeitung eines vorgebbaren Steuerprogramms für das Steuerfluidventil und das Arbeitsfluidventil ausgebildet sind. Die Steuereinrichtung kann ihrerseits für eine Kommunikation mit einer übergeordneten Steueranordnung, insbesondere einer speicherprogrammierbaren Steuerung, vorgesehen sein und hierfür eine geeignete Kommunikationsschnittstelle, insbesondere eine Multipol- Schnittstelle oder eine Busschnittstelle, umfassen.

Eine bevorzugte Ausführungsform der Erfindung sieht vor, dass am Grundkörper ein Steuerfluid-Drucksensor angeordnet ist, der elektrisch mit der Steuereinrichtung verbunden ist und der für eine Erfassung eines Steuerfluiddrucks ausgebildet ist. Der Steuerfluid-Drucksensor kann über eine Fluidleitung fluidisch kommunizierend mit der Steuerfluidleitung verbunden sein oder in der Steuerfluidleitung angeordnet sein oder benachbart zur Steuerfluidmündung angeordnet sein und dient zur Erfassung des Steuerfluiddrucks, der bei Kopplung der Betätigungseinrichtung mit einer Membranpumpeneinrichtung auf die Pumpmembran aufgebracht werden kann. Anhand des gemessenen Steuerfluiddrucks und/oder einer zeitlichen Ableitung des gemessenen Steuerfluiddrucks können in der Steuereinrichtung Rückschlüsse darauf gezogen werden, in welcher Weise die Pumpmembran deformiert ist und ein Fördervorgang für das mit Hilfe der Membranpumpeneinrichtung zu fördernde Fluid abläuft.

Bei einer Weiterbildung der Erfindung ist vorgesehen, dass der Arbeitsfluidleitung ein Arbeitsfluid-Drucksensor angeordnet ist, der elektrisch mit der Steuereinrichtung verbunden ist und der für eine Erfassung eines Arbeitsfluiddrucks ausgebildet ist. Der Arbeitsfluid-Drucksensor kann über eine Sensorleitung fluidisch kommunizierend mit der Arbeitsfluidleitung verbunden sein oder in der Arbeitsfluidleitung angeordnet sein. Ein Sensorsignal des Arbeitsfluid-Drucksensors wird entweder im Arbeitsfluidventil und/oder in der Steuereinrichtung verarbeitet und dient insbesondere zur Druckregelung des Arbeitsfluiddrucks, der an den Fluidaktor bereitgestellt wird. Bei einer Bereitstellung des Sensorsignals des Arbeitsfluid-Drucksensors an die Steuereinrichtung kann unter zusätzlicher Einbeziehung des Sensorsignals des Steuerfluid-Drucksensors eine besonders exakte Ansteuerung des Fluidaktors erfolgen, der beispielhaft zur Betätigung eines Membranventils der Membranpumpeneinrichtung ausgebildet ist.

In weiterer Ausgestaltung der Erfindung ist vorgesehen, dass an der Montagefläche eine konkave Steuerkammer als Bewegungsraum für eine Pumpmembran ausgebildet ist und dass die Steuerfluidmündung an einer Oberfläche der Steuerkammer aus- mündet. Mit der konkav ausgebildeten Steuerkammer wird ein vorteilhafter Pumpenhub für die an der Membranpumpeneinrichtung ausgebildete Membranpumpe ermöglicht, da die Membran der Membranpumpe zusätzlich zu einem ohnehin möglichen konkaven Deformationszustand bei Druckbeaufschlagung der Steuerfluidleitung auch in einen konvexen Deformationszustand gebracht werden kann, der bei einer Unterdruckbeaufschlagung der Steuerkammer auftreten kann. Vorzugsweise ist in der Steuerkammer ein fluiddurchlässiger Formkörper vorgesehen, der beabstandet von der Oberfläche der Steuerkammer angeordnet ist und der bei einer Unterdruckbeaufschlagung der Membran der Membranpumpe als Anlagefläche für die Membran dient. Ein Raumvolumen zwischen der Oberfläche der Steuerkammer und einer der Membran zugewandten Anlagefläche des Formkörpers dient als Arbeitsraum für das an der Steuerfluidmündung bereitstellbare Steuerfluid, das beispielsweise nach einer Unterdruckbeaufschlagung der Steuerkammer und einer dadurch bedingten flächigen Anlage der Membran an der Anlagefläche für einen Austragvorgang der Membranpumpe genutzt werden soll.

Bevorzugt ist im Grundkörper eine Unterdruckleitung ausgebildet, die sich von einem Unterdruckanschluss bis zur Steuerkammer oder zur Steuerfluidleitung erstreckt und in der ein Unterdruckventil angeordnet ist, das für eine Beeinflussung eines Querschnitts der Unterdruckleitung ausgebildet ist, wo- bei das Unterdruckventil elektrisch mit der Steuereinrichtung verbunden ist. In Abhängigkeit von der Gestaltung der Steuerkammer und des darin gegebenenfalls aufgenommenen Formkörpers kann wahlweise vorgesehen sein, dass die Unterdruckleitung unmittelbar an der Oberfläche der Steuerkammer ausmündet oder fluidisch kommunizierend mit der Steuerfluidleitung verbunden ist. Durch eine zyklische Wechselbeaufschlagung der Steuerkammer und der daran ankoppelbaren Membran der Membranpumpeneinrichtung wird eine Pumpbewegung für die Membran zwischen einem konkaven Deformationszustand bei Druckbeaufschlagung der Steuerfluidleitung und konvexen Deformationszustand und damit ein maximaler Pumpenhub ermöglicht. Bei der Unterdruckleitung handelt es sich ebenfalls um eine Steuerfluidleitung.

Vorteilhaft ist es, wenn die Arbeitsfluidleitung fluidisch kommunizierend mit dem Steuerfluidanschluss verbunden ist.

Bei einer vorteilhaften Weiterbildung der Erfindung ist vorgesehen, dass die Steuereinrichtung einen Beobachteralgorithmus umfasst, der für eine Ermittlung eines Fluidmassenstroms in einer Membranpumpeneinrichtung anhand des Drucksignals des Steuerfluid-Drucksensors ausgebildet ist. Die Aufgabe des Beobachteralgorithmus besteht darin, den Fluidmassenstrom in der Membranpumpeneinrichtung zu bestimmen, ohne dass hierfür eine Sensoreinrichtung unmittelbar in der Membranpumpeneinrichtung integriert ist. Der Beobachteralgorithmus kann beispielsweise als Gleichungssystem oder als neuronales Netzwerk ausgebildet sein. Vorzugsweise wird der Beobachteralgorithmus mit Hilfe einer Referenzanordnung, die die Betätigungseinrichtung und die daran angebrachten Membranpumpeneinrichtung sowie einen externen, präzisen Durchflussmesser umfasst, durch Abgleich der Signale des Steuerfluid-Drucksensors und des externen Durchflussmessers kalibriert.

Bei einer besonders bevorzugten Ausführungsform ist am Grundkörper eine erste Stelleinrichtung mit einem ersten Arbeitsfluidventil und eine zweite Stelleinrichtung mit einem zweiten Arbeitsfluidventil angeordnet sind, wobei das erste Arbeitsfluidventil als Schaltventil ausgebildet ist und wobei das zweite Arbeitsfluidventil als Proportionalventil, insbesondere als Druckregelventil, ausgebildet ist. Hierdurch kann die Betätigungseinrichtung bei angekoppelter Membranpumpeneinrichtung sowohl Einfluss auf einen Fluidzustrom in die Pumpkammer als auch Einfluss auf einen Fluidabstrom aus der Pumpkammer nehmen. Vorzugsweise ist die Steuereinrichtung derart ausgebildet, dass sie eine zyklisch wiederkehrende, gegensinnige Ansteuerung der beiden Stelleinrichtungen in Abhängigkeit von einer Bereitstellung von Steuerfluid an die Steuerkammer ermöglicht. Bevorzugt ist vorgesehen, dass die erste Stelleinrichtung in einem gesteuerten Betrieb betrieben wird, während die zweite Stelleinrichtung in einem geregelten Betrieb, insbesondere in Abhängigkeit von einem Sensorsignal eines dem zweiten Arbeitsfluidventil zugeordneten Arbeitsfluid-Drucksensors, betrieben wird. In dem geregelten Betrieb wirkt das zweite Arbeitsfluidventil als Druckregelventil. Hierdurch kann insbesondere eine Einhaltung eines engen begrenzten Durchflussintervalls für einen Fluidmassenstrom, der von der Membranpumpeneinrichtung während eines Pumpenhubs gefördert wird, gewährleistet werden.

Bei dem erfindungsgemäßen Verfahren zum Betreiben einer Betätigungseinrichtung erfolgt eine Ansteuerung des Arbeitsfluidventils durch die Steuereinrichtung, die mit einem Beobachteralgorithmus einen Fluidmassenstrom in einer Membranpumpeneinrichtung anhand der Drucksignale eines Steuerfluid-Drucksensors ermittelt und die einen Drucksollwert für das Arbeitsfluidventil in Abhängigkeit vom ermittelten Fluidmassenstrom bestimmt, um während einer Austragphase der Membranpumpeneinrichtung einen konstanten Fluidmassenstrom zu gewährleisten.

Bei einer Weiterbildung des Verfahrens ist vorgesehen, dass das die Steuereinrichtung das Steuerfluidventil und das Unterdruckventil während einer Austragphase der Membranpumpeneinrichtung derart ansteuert, dass die Steuerfluidleitung und die Unterdruckleitung gesperrt sind. Somit erfolgt der Austrag des Fluids aus der Pumpkammer der Membranpumpe ohne einen weiteren Zustrom von Steuerfluid in die Steuerkammer, sondern vielmehr aufgrund des bei Beginn der Austragphase in der Steuerkammer vorliegenden Steuerfluiddrucks in Verbindung mit der inneren Spannung in der deformierten Membran der Membranpumpe. Somit kann der Steuerfluiddruck als Maß für die Volumenänderung in der Steuerkammer und somit indirekt als Maß für den Fluidmassenstrom aus der Pumpkammer der Membranpumpe genutzt werden.

Die erfindungsgemäße Membranpumpeneinrichtung, die von der erfindungsgemäßen Betätigungseinrichtung mechanisch angesteuert werden kann, weist vorzugsweise einen Pumpenkammerkörper auf, in dem eine Ausnehmung ausgebildet ist, die zur Bildung einer Pumpenkammer von einer elastischen Membran verschlossen ist. Darüber hinaus umfasst die Membranpumpeneinrichtung einen Zustrompfad, der einen Eingangsanschluss mit einer Einlassöffnung der Pumpenkammer verbindet, und einen Abstrompfad, der eine Auslassöffnung der Pumpenkammer mit einem Ausgangsanschluss verbindet. In dem Zustrompfad ist ein Einlassventil und in dem Abstrompfad ist ein Auslassventil vorgesehen, um die Flüssigkeitsströmung in dem Zustrom- bzw. Abstrompfad beeinflussen zu können.

Die Membranpumpeneinrichtung oder ein Teil der Membranpumpeneinrichtung kann als eine zur einmaligen Verwendung bestimmte Kassette ausgebildet, die für die Verwendung mit der Betätigungseinrichtung der Membranpumpe bestimmt ist. Die Membranpumpeneinrichtung kann als eine an eine Montagefläche der Betätigungseinrichtung ankoppelbare Einrichtung ausgebildet sein. Membranpumpeneinrichtung und Betätigungseinrichtung können aber auch im Gebrauch nicht voneinander trennbare Einrichtungen sein.

Die erfindungsgemäße Membranpumpe umfasst die erfindungsgemäße Betätigungseinrichtung und die erfindungsgemäße Pumpeneinrichtung.

Die Membranpumpe kann vorteilhafterweise in der Medizintechnik Verwendung finden. Vorteile ergeben sich aber auch bei einer Verwendung in anderen Bereichen der Technik. Eine besonders bevorzugte Verwendung ist der Einsatz der Membranpumpe in einer Blutbehandlungsvorrichtung, insbesondere Dialysevorrichtung, mit einem Behältnis zur Bereitstellung einer medizinischen Flüssigkeit, insbesondere einer Antikoagulations-Lösung, die mit einer besonders hohen Fördergenauigkeit bei einer relativ geringen Flussrate gefördert werden soll.

Nachfolgend wird ein Ausführungsbeispiel einer Membranpumpe, die eine Pumpeneinrichtung und eine Betätigungseinrichtung umfasst, im Einzelnen beschrieben.

Es zeigen:
- Fig. 1: eine Schnittdarstellung eines Teils einer Betätigungseinrichtung mit einer Steuerkammer und einer Stelleinrichtung,
- Fig. 2: eine Schnittdarstellung einer Membranpumpeneinrichtung,
- Fig. 3: ein schematisches Schaltbild der Betätigungseinrichtung gemäß der Fig. 1,
- Fig. 4: ein Steuerdiagramm für die Betätigungseinrichtung gemäß der Fig. 1.

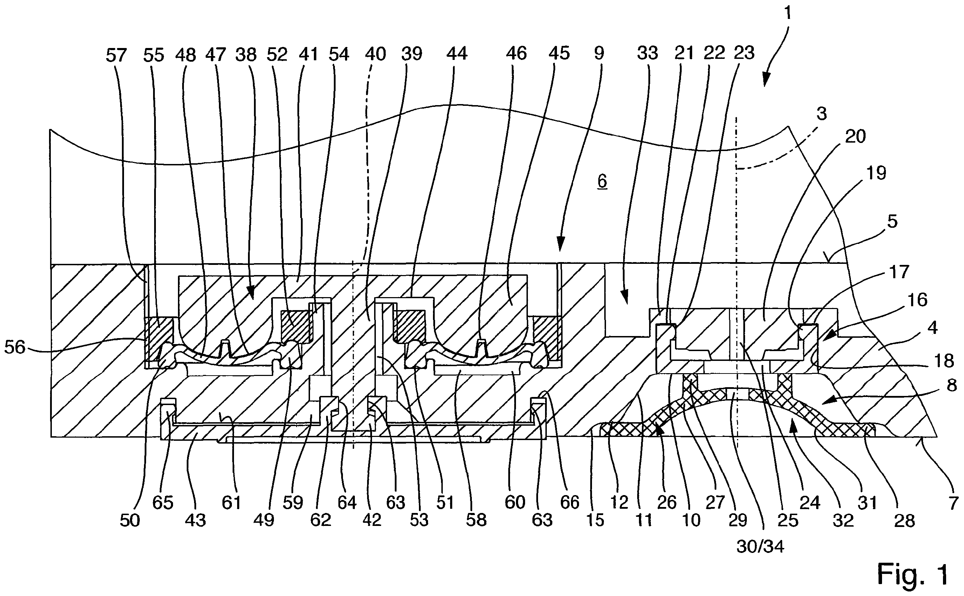

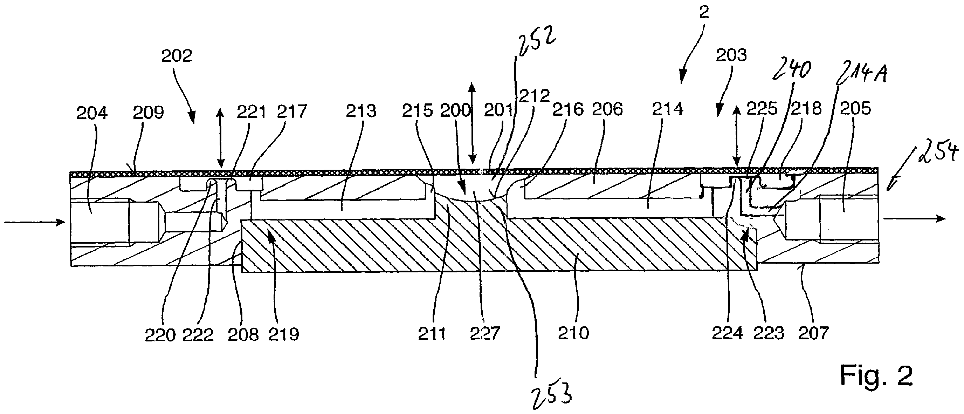

Die in der Fig. 1 dargestellte Betätigungseinrichtung 1 ist für eine mechanische Ansteuerung einer in Fig. 2 dargestellten Membranpumpeneinrichtung 2 vorgesehen, wobei ein nicht dargestellter mechanischer Verbund aus der Betätigungseinrichtung 1 und der Membranpumpeneinrichtung 2 eine Fluidförderung eines vorzugsweise flüssigen Fluids bei vollständiger Trennung des zu fördernden Fluids von der Betätigungseinrichtung 1 ermöglicht.

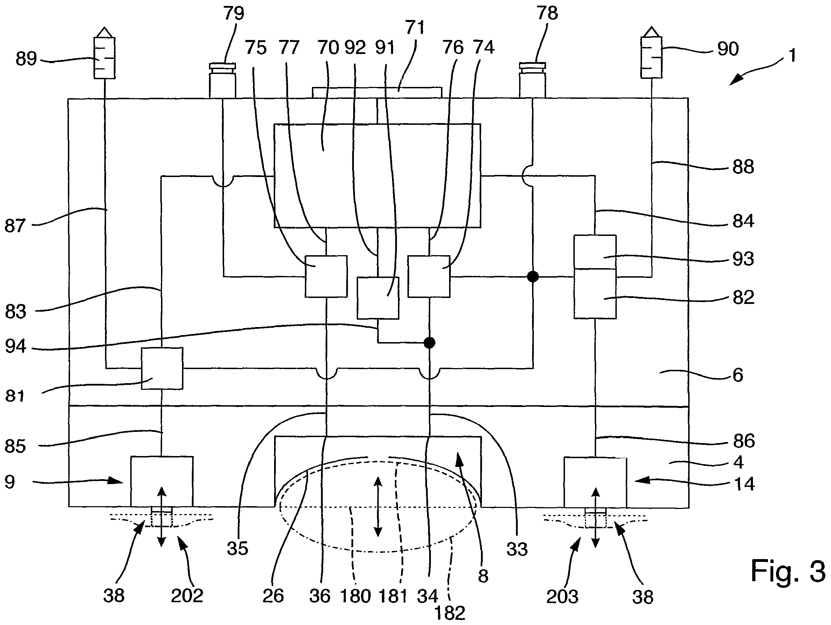

Die Betätigungseinrichtung 1 ist gemäß der Darstellung der Fig. 1 rein exemplarisch spiegelsymmetrisch zu einer Spiegelebene ausgebildet, die senkrecht zur Darstellungsebene der Fig. 1 ausgerichtet ist und die eine Mittelachse 3 umfasst. Die Betätigungseinrichtung 1 umfasst einen Grundkörper 4, der beispielhaft quaderförmig ausgebildet ist und der insbesondere als Kunststoffspritzgussteil hergestellt sein kann. An einer beispielhaft eben ausgebildeten Oberseite 5 des Grundkörpers 4 ist eine Steuerbaugruppe 6 angebracht, die nachstehend im Zusammenhang mit der Fig. 3 näher beschrieben wird. Eine exemplarisch zumindest im Wesentlichen eben ausgebildete Unterseite des Grundkörpers 4 dient als Montagefläche 7 für die in Fig. 2 dargestellte und ebenfalls nachstehend näher beschriebene Membranpumpeneinrichtung 2.

Wie der teilweise geschnittenen Darstellung der Fig. 1 entnommen werden kann, sind im Grundkörper 4 eine Steuerkammer 8 sowie eine Stelleinrichtung 9 angeordnet. Dabei ist die Steuerkammer 8 für eine Betätigung einer Membran 201 einer Membranpumpe 200, die in der Membranpumpeneinrichtung 2 ausgebildet ist, vorgesehen. Die Stelleinrichtung 9 dient zur Ansteuerung eines Membranventils 202, 203, das ebenfalls in der Membranpumpeneinrichtung 2 ausgebildet ist.

Die Steuerkammer 8 ist beispielhaft als rotationssymmetrische Ausnehmung in der Montagefläche 7 ausgebildet, wobei die Mittelachse 3 als Rotationsachse für ein Profil der Steuerkammer 8 vorgesehen ist. Die Steuerkammer 8 ist im Wesentlichen mit einem napfförmigen Querschnitt versehen und weist einen kreisrunden Bodenbereich 10, einen konusabschnittsförmigen Wandbereich 11, eine kreisringförmige Kontaktfläche 12 und einen daran angrenzenden, konusabschnittsförmigen Auslaufbereich 15 auf. Der Bodenbereich 10 wird beispielhaft von einer Oberfläche eines kreisringförmigen Dichtelements 16 gebildet, das in einer an die Steuerkammer 8 angrenzenden Ausnehmung 17 eingesetzt ist und das bezogen auf die Mittelachse 3 ein L-förmiges Profil aufweist. Das Dichtelement 16 liegt mit einer kreiszylindrischen Außenoberfläche 18 flächig an einer gegenüberliegenden, nicht bezeichneten inneren Oberfläche der Ausnehmung 17 an und weist an einem kürzeren L-Schenkel einen radial nach innen weisenden Ringbund 19 auf, der für eine abdichtende Anlage an einer Außenoberfläche eines rotationssymmetrisch zur Mittelachse 3 ausgebildeten Kanalteils 20 vorgesehen ist. Um eine eindeutige Platzierung des Dichtelements 16 und des Kanalteils 20 zu gewährleisten weist die Ausnehmung 17 einen radial nach innen vorspringenden Ringbund 21 auf, an dem eine axiale, kreisringförmige Stirnseite 22 des Dichtelement 16 formschlüssig anliegt. Das Kanalteil 20 liegt seinerseits mit einer Stufe 23 an der Stirnseite 22 an. Sowohl das Dichtelement 16 als auch das Kanalteil 20 sind jeweils von einer zentrischen Ausnehmung 24, 25 durchsetzt, die als Bestandteil einer Steuerfluidleitung 33 anzusehen sind.

In der Steuerkammer 8 ist ein Formkörper 26 aufgenommen, der rein exemplarisch rotationssymmetrisch zur Mittelachse 3 ausgebildet ist und umfasst einen im Wesentlichen als Kugelschalenabschnitt ausgeführten Hauptkörper 27, einen hiervon radial nach außen abragenden Ringbund 28 und einen koaxial zur Mittelachse 3 ausgerichteten Stützring 29. Der Hauptkörper 27 ist von einer zentral angeordneten Ausnehmung 30 durchsetzt und bildet an einer dem Stützring 29 abgewandten Oberfläche eine als Kugelkalotte geformte Anlagefläche 31 aus.

Der Ringbund 28 liegt an der Kontaktfläche 12 der Steuerkammer 8 an, während der Stützring 29 stirnseitig am Bodenbereich 10 der Steuerkammer 8 abgestützt ist. Die Anlagefläche 31 dient als Begrenzung für einen Raumabschnitt, der als Membranarbeitsbereich 32 bezeichnet wird und begrenzt bei Anbringung der Membranpumpeneinrichtung 2 an der Betätigungseinrichtung 1 eine Deformation der Membran 201 der Membranpumpe 200. Die Ausnehmung 30 im Formkörper 26 dient als Steuerfluidmündung 34.

Benachbart zur Steuerkammer 8 ist im Grundkörper 4 die Stelleinrichtung 9 angeordnet, bei der es sich beispielhaft um einen fluidisch betreibbaren Linearaktor handelt. Exemplarisch umfasst die Stelleinrichtung 9 ein linearbeweglich in einer Ausnehmung 37 aufgenommenes Stellglied 38, das zur Betätigung des Membranventils 202, 203 der Membranpumpeneinrichtung 2 vorgesehen ist. Das Stellglied 38 wird beispielhaft ohne eine Bereitstellung von druckbeaufschlagtem Fluid durch Einwirkung einer nicht dargestellten Feder in eine nicht dargestellte Ruheposition verlagert. Gemäß der Darstellung der Fig. 1 befindet sich das Stellglied 38 durch Bereitstellung von druckbeaufschlagtem Fluid in einer Funktionsposition. Das Stellglied 38 umfasst einen zylindrischen Stößel 39, dessen Längsachse 40 quer zur Montagefläche 7 ausgerichtet ist. Der Stößel 39 ist mit einem hinteren Endbereich an einer Verbindungsplatte 41 angebracht und ist mit einem vorderen Endbereich 42 an einer tellerförmig ausgebildeten Dichtmembran 43 festgelegt. Die Verbindungsplatte 41 ist rein exemplarisch kreisscheibenförmig ausgebildet und weist an einer Vorderseite 44, an der der Stößel 39 mittig angebracht ist, in einem radial außen liegenden Bereich einen kreisringförmigen Stützring 45 auf, der in die gleiche Richtung wie der Stößel 39 abragt. Wie aus der Darstellung der Fig. 1 entnommen werden kann, weist der Stützring 45 einen U-förmigen Querschnitt mit einer zentral angeordneten, umlaufenden Nut 46 auf und liegt mit einer der Vorderseite 44 abgewandten, konvex gekrümmten Stützfläche 47 an einem Dichtring 48 an.

Der Dichtring 48 ist koaxial zum Stößel 39 und rotationssymmetrisch zur Längsachse 40 ausgebildet und weist radial innenliegend sowie radial außenliegend jeweils einen ringförmigen Dichtwulst 49, 50 auf. Der innenliegende Dichtwulst 49 ist in einer nutförmigen, umlaufenden Vertiefung 51 im Grundkörper 4 aufgenommen und wird rein exemplarisch von einem Schraubring 52, der auf einen koaxial zur Längsachse 40 ausgebildeten und mit einer Ausnehmung 53 für den Stößel 39 versehenen Schraubstutzen 54 des Grundkörpers 4 aufgeschraubt ist, am Grundkörper 4 festgelegt. In gleicher Weise wird der außen liegende Dichtwulst 50 von einem Schraubring 55, der mit einem Außengewinde 56 in ein am Grundkörper 4 ausgebildetes Innengewinde 57 eingeschraubt ist, ebenfalls am Grundkörper 4 festgelegt.

In einem Bereich zwischen dem inneren Dichtwulst 49 und dem äußeren Dichtwulst 50 ist gegenüberliegend zum Stützring 45 im Grundkörper 4 eine ringförmige Ausnehmung 60 eingebracht, die eine Linearbewegung des Stellglieds 38 entlang der Längsachse 40 und gemäß der Darstellung der Fig. 1 nach unten ermöglicht. Der abdichtend am Grundkörper 4 festgelegte Dichtring 48 bestimmt zusammen mit der ringförmigen Ausnehmung 60 einen Arbeitsraum, der einen Endbereich eines Arbeitsfluidkanals 58 darstellt und mit druckbeaufschlagtem Fluid versorgt werden kann. Bei einer solchen Versorgung dieses Arbeitsraums wird das Stellglied aufgrund einer elastischen Deformation des Dichtrings 48 aus einer nicht dargestellten Ruheposition in die Funktionsposition gemäß der Fig. 1 verlagert. Hier-durch wird auch die Dichtmembran 43 deformiert, die aus einer von der Montagefläche 7 abragenden Stellung in die zumindest im Wesentlichen oberflächenbündige Stellung gemäß der Fig. 1 gebracht werden kann. Dabei ist vorgesehen, dass die Dichtmembran 43 in der Ruhestellung an dem jeweils zugeordneten Membranventil 202, 203 der Membranpumpeneinrichtung 2 anliegt, um das Membranventil 202, 203 aus einer Öffnungsstellung in eine Schließstellung zu bringen. Bei der Beaufschlagung des Arbeitsraums mit druckbeaufschlagtem Fluid findet durch die damit verbundene Verlagerung des Stellglieds 38 in die Funktionsposition gemäß der Fig. 1 eine Öffnungsbewegung 30 für das jeweils zugeordnete Membranventil 202, 203 der Membranpumpeneinrichtung 2 statt, wie dies schematisch auch in der Fig. 3 dargestellt ist.

Die Dichtmembran 43 weist an einer dem Stößel 39 zugewandten Oberseite 61 einen Ringbund 62 auf, der mit einem radial nach innen weisenden Vorsprung 63 versehen ist, der formschlüssig in eine am vorderen Endbereich 42 ausgebildete Ringnut 64 eingreift. Durch diese formschlüssige Verbindung zwischen Stößel 39 und Dichtmembran 43 wird eine bidirektionale Krafteinleitung vom Stößel 39 auf die Dichtmembran 43 und umgekehrt ermöglicht. In einem radial außenliegenden Umfangsbereich der Dichtmembran 43 ist diese mit einem koaxial abragenden, einstückig angeformten Haltering 65 versehen, der in einer im Grundkörper 4 koaxial zur Längsachse 40 nutartig eingebrachte Ringnut 66 kraftschlüssig festgelegt ist, so das die Dichtmembran 43 eine fluidische Trennung zwischen dem Bewegungsraum 59 und einer Umgebung der Betätigungseinrichtung gewährleistet.

In der Fig. 2 ist eine rein schematische Schnittdarstellung einer Membranpumpeneinrichtung 2 gezeigt, die für eine Ankopplung an die Betätigungseinrichtung 1 ausgebildet ist und die für eine Förderung eines Fluids, das an einem Eingangsanschluss 204 bereitgestellt wird, zu einem Ausgangsanschluss 205, genutzt werden kann. Beispielhaft umfasst die Membranpumpeneinrichtung 2 eine quaderförmige Grundplatte 206, die von einer Unterseite 207 mit einer Ausnehmung 208 versehen ist. Die Ausnehmung 208 ist mit einer von der Unterseite 207 montierten Verschlussplatte 210 zumindest weitestgehend ausgefüllt, die in einem der Unterseite 207 abgewandten zentralen Bereich einen Stützvorsprung 211 aufweist, der sich in Richtung der Oberseite 209 erstreckt und der an einer der Unterseite 207 abgewandten Stirnseite 212 eine konkave, insbesondere kugelkalottenförmige Gestalt aufweist. Dadurch wird eine Pumpenkammer 252 in einem Pumpenkammerkörper 253 gebildet, der Teil des Gehäusekörpers 254 der Pumpeneinrichtung 2 ist. Die Ausnehmung 208 in der Grundplatte 206 ist derart ausgebildet, dass nach Montage der Verschlussplatte 210 in die Grundplatte 206 ein nutförmiger Zustromkanal 213 sowie ein nutförmiger Abstromkanal 214 gebildet werden, die jeweils an Mündungsöffnungen 215, 216 benachbart zum Stützvorsprung 211 in Richtung der Oberseite 209 ausmünden. An einem dem Stützvorsprung 211 abgewandten Endbereich mündet der Zustromkanal 213 in einen beispielhaft zylindrisch ausgebildeten Ventilraum 217 eines ersten Membranventils 202, in gleicher Weise mündet der Abstromkanal 214 an einem dem Stützvorsprung 211 abgewandten Endbereich in einen beispielhaft zylindrisch ausgebildeten Ventilraum 218 eines zweiten Membranventils 203.

Das erste Membranventil 202 ist als Einlassventil für eine Beeinflussung eines Zustrompfads 219, der den Eingangsanschluss 204, den Ventilraum 217 und den Zustromkanal 213 umfasst, vorgesehen. Hierzu ist im Ventilraum 217 ein ringförmig ausgebildeter Ventilsitz 220 angeordnet, dessen Stirnseite 221 beabstandet zur Oberseite 209 der Grundplatte 206 angeordnet ist und der zentral von einem Ventilkanal 222 durchsetzt ist. Demgegenüber ist die fluidisch kommunizierende Verbindung zwischen dem Ventilraum 217 und dem Zustromkanal 213 in einem radial außenliegenden Bereich des Ventilraums 217 verwirklicht.

Das zweite Membranventil 203 ist als Auslassventil für eine Beeinflussung eines Abstrompfads 223, der den Ausgangsanschluss 205, den Ventilraum 218 und den Abstromkanal 214 umfasst, vorgesehen. Hierzu ist im Ventilraum 218 ein ringförmig ausgebildeter Ventilsitz 224 angeordnet, der zentral von einem Ventilkanal 240 durchsetzt ist. Die Stirnseite des Ventilsitzes 224 ist beabstandet zur Oberseite 209 der Grundplatte 206 angeordnet. Der Ventilsitz 224 wird von dem Ventilraum konzentrisch umschlossen, wobei der Abstromkanal 214 die Mündungsöffnung 216 der Membranpumpe 200, d. h. deren Auslassöffnung, mit dem Ventilraum 218 verbindet. Die fluidisch kommunizierende Verbindung zwischen dem Ventilraum 218 und dem Ausgangsanschluss 205 ist in einem radial außenliegenden Bereich des Ventilraums 218 verwirklicht. Der Abstrompfad 223 umfasst neben dem zu dem Auslassventil 203 führenden ersten Abstromkanal 214 einen zweiten von dem Auslassventil 203 abgehenden Abstromkanal 214A, der an den Ventilkanal 240 angeschlossen ist. Der zweite Abstromkanal 214A verbindet den Ventilkanal 240 mit dem Ausgangsanschluss 205.

Wenn der Abstrompfad einen ersten Abstromkanal umfasst, der die Auslassöffnung der Pumpenkammer mit einer Einlassöffnung des Ventilraums des Auslassventils verbindet, und einen zweiten Abstromkanal umfasst, der den Auslassventilkanal mit dem Ausgangsanschluss verbindet, wobei die Querschnittsfläche des Auslassventilsitzes kleiner als die Querschnittsfläche des den Auslassventilsitz umschließenden Bereichs des Auslassventilraums ist, kann das Verhalten der Regelung der Flüssigkeitsströmung noch verbessert werden. Es hat sich gezeigt, dass die Regelung bei einer derartigen Anordnung nicht zum Schwingen neigt. Grundsätzlich ist aber auch möglich, dass der erste Abstromkanal die Auslassöffnung der Pumpenkammer mit dem Auslassventilkanal und der zweite Abstromkanal eine Auslassöffnung des Ventilraums des Auslassventils mit dem Ausgangsanschluss verbindet.

Rein exemplarisch ist die gesamte Oberseite 209 der Grundplatte 206 mit einer gummielastischen Membran 201 überzogen, die beispielsweise stoffschlüssig an der Oberseite 209 festgelegt ist und somit eine fluidische Trennung zwischen den Ventilräumen 217, 218 und einer Umgebung der Membranpumpeneinrichtung 2 gewährleistet. Ferner dient die Membran 201 auch zur Abtrennung eines Pumpraums 227, der von der Ausnehmung 208 sowie vom Stützvorsprung 211 und der Membran 201 berandet wird, von der Umgebung der Membranpumpeneinrichtung 2.

Bei einer Anbringung der Membranpumpeneinrichtung 2 an der Betätigungseinrichtung 1 kann mit Hilfe der in der Fig. 1 dargestellten ersten Stelleinrichtung 9 beispielsweise eine Deformation der Membran 201 über dem Ventilsitz 220 vorgenommen werden, um dadurch die Membran 201 lokal abdichtend auf die Stirnseite 221 des Ventilsitzes 220 zu pressen und damit eine fluidisch kommunizierende Verbindung zwischen dem Eingangsanschluss 204 und dem Zustromkanal 213 zu unterbrechen. Ferner kann mit Hilfe einer spiegelbildlich zu der in Fig. 1 dargestellten zweiten Stelleinrichtung 9 (14), die aus Gründen der Übersichtlichkeit in der Fig. 1 nicht gezeigt ist, eine Deformation der Membran 201 über dem Ventilsitz 224 vorgenommen werden, um dadurch die Membran 201 lokal abdichtend auf die Stirnseite 225 des Ventilsitzes 224 zu pressen und damit eine fluidisch kommunizierende Verbindung zwischen dem ersten Ab stromkanal 214 und dem Ausgangsanschluss 205 zu unterbrechen.

Darüber hinaus kann die Membran 201 im Bereich des Pumpraums 227 oberhalb des Stützvorsprungs 211 durch geeignete Beaufschlagung der Ausnehmung 30 mit Steuerfluid oder einem Unterdruck aus der in Fig. 2 dargestellten Neutralstellung in eine rein schematisch in der Fig. 3 dargestellte konvexe Ansaugkonfiguration oder in eine rein schematisch in der Fig. 3 dargestellte konkave Austragkonfiguration gebracht werden.

Die in der Fig. 3 dargestellte Betätigungseinrichtung 1 ist, wie bereits obenstehend ausgeführt wurde, rein exemplarisch in den Grundkörper 4 und die Steuerbaugruppe 6 aufgeteilt, wobei die im Grundkörper 4 enthaltenen Komponenten bereits im Zusammenhang mit der Fig. 1 vorstehend umfassend beschrieben wurden.

Die Steuerbaugruppe 6 enthält eine Steuereinrichtung 70, die beispielsweise als Mikroprozessor oder Mikrocontroller ausgebildet sein kann und die zur Abarbeitung eines vorgebbaren Ablaufprogramms ausgebildet ist. Exemplarisch ist vorgesehen, dass die Steuereinrichtung 70 über eine Schnittstelle 71 in elektrische Verbindung mit einer nicht dargestellten übergeordneten Steuerungseinrichtung, bei der es sich beispielsweise um eine speicherprogrammierbare Steuerung handeln kann, gebracht werden kann, so dass die übergeordnete Steuerungseinrichtung Steuerbefehl an die Steuereinrichtung 70 bereitstellen kann. Alternativ ist vorgesehen, dass die Steuereinrichtung 70 ohne externe Befehle autark arbeiten kann und die nachstehend näher beschriebenen Komponenten in geeigneter Weise ansteuert. Bei diesen Komponenten handelt es sich zum einen um eine Steuerfluidanordnung 72 zur Bereitstellung von Steuerfluid an die Steuerkammer 8 und zum anderen um eine Arbeitsfluidanordnung 73 zur Bereitstellung von Arbeitsfluid an die beiden schematisch eingezeichneten Stelleinrichtungen 9, 14, die als Einlass-Stelleinrichtung 9 und Auslass-Stelleinrichtung 14 bezeichnet werden.

Die Steuerfluidanordnung 72 umfasst ein erstes Steuerfluidventil 74 und ein zweites Steuerfluidventil 75, die beispielhaft jeweils als Magnetventile ausgebildet sein können und die elektrisch über Steuerleitungen 76, 77 mit der Steuereinrichtung 70 verbunden sind. Das erste Steuerfluidventil 74 steht über die erste Steuerfluidleitung 33, die in der Steuerkammer 8 mit einer ersten Steuerfluidmündung 34 ausmündet, in fluidisch kommunizierender Verbindung. Ferner ist das erste Steuerfluidventil 74 fluidisch kommunizierend mit einem ersten Steuerfluidanschluss 78 verbunden und ermöglicht eine wahlweise Freigabe oder Blockierung einer fluidisch kommunizierenden Verbindung zwischen dem ersten Steuerfluidanschluss 78 und der ersten Steuerfluidmündung 34.

Das zweite Steuerfluidventil 75 steht über eine zweite Steuerfluidleitung 35, die in der Steuerkammer 8 mit einer zweiten Steuerfluidmündung 36 ausmündet, in fluidisch kommunizierender Verbindung. Ferner ist das zweite Steuerfluidventil 75 fluidisch kommunizierend mit einem zweiten Steuerfluidanschluss 79 verbunden und ermöglicht eine wahlweise Freigabe oder Blockierung einer fluidisch kommunizierenden Verbindung zwischen dem zweiten Steuerfluidanschluss 79 und der zweiten Steuerfluidmündung 36.

Vorzugsweise ist vorgesehen, dass am ersten Steuerfluidanschluss 78 eine nicht dargestellte Druckluftquelle angeschlossen ist, während am zweiten Steuerfluidanschluss 79 eine nicht dargestellte Unterdruckquelle angeschlossen ist. Somit kann bei einer Anbringung der in Fig. 2 dargestellten Membranpumpeneinrichtung 2 an der in Fig. 3 dargestellten Betätigungseinrichtung 1 durch geeignete, vorzugsweise wechselweise, insbesondere zyklisch wiederkehrende, Ansteuerung der beiden Steuerfluidventile 74 und 75 eine Abfolge einer Überdruckbeaufschlagung und einer Unterdruckbeaufschlagung für die Steuerkammer 8 bewirkt werden, wodurch die Membran 201 der Membranpumpe 200 aus der Neutralstellung 180 zunächst in eine konvex gewölbte Ansaugstellung 181 und anschließend in eine konkav gewölbte Austragstellung 182 gebracht werden kann. Durch diese wechselnde Deformation der Membran 201 der Membranpumpe 200 wird ein Förderhub für ein in der Membranpumpe 200 aufgenommenes Fluid bewirkt, so dass dieses bei geeigneter Ansteuerung der Membranventile 202 und 203 vom Eingangsanschluss 204 zum Ausgangsanschluss 200 gefördert werden kann. Um diese Ansteuerung der beiden Membranventile 202 und 203 bewirken zu können, sind die Einlass- und Auslass-Stelleinrichtungen 9 und 14 vorgesehen, die jeweils mit den zugeordneten Einlass- und Auslass-Stellgliedern 38 auf die gegenüberliegenden Abschnitte der Membran 201 einwirken können, um wahlweise eine abdichtende Anlage der Membran 201 am jeweiligen Ventilsitz 220, 224 oder eine Freigabe des jeweiligen Ventilsitzes 220, 224 zu ermöglichen.

Zu diesem Zweck umfasst die Einlass-Stelleinrichtung 9, die rein exemplarisch für eine Beeinflussung des dem Zustromkanal 213 zugeordneten Membranventils 202 vorgesehen ist, ein erstes Arbeitsfluidventil 81, das über eine Steuerleitung 83 mit der Steuereinrichtung 70 verbunden ist, um eine elektrische Ansteuerung des Arbeitsfluidventils 81 zu ermöglichen. Ferner ist das Arbeitsfluidventil 81 fluidisch mit dem ersten Steuerfluidanschluss 78 verbunden und über eine erste Arbeitsfluidleitung 85 mit der ersten Stelleinrichtung 9 fluidisch kommunizierend gekoppelt. Dementsprechend kann das Arbeitsfluidventil 81, bei dem es sich insbesondere um ein 2/2- Wege-Magnetventil handeln kann, wahlweise eine Versorgung der Einlass-Stelleinrichtung 9 mit druckbeaufschlagtem Arbeitsfluid oder eine Entlüftung der Stelleinrichtung 9 bewirken, wobei die Entlüftung über einen ersten Entlüftungskanal 87 bis zu einem ersten Schalldämpfer 89 erfolgt. Wie aus der Darstellung der Fig. 1 hervorgeht, führt eine Beaufschlagung der ersten Stelleinrichtung 9 mit druckbeaufschlagtem Fluid zu einer Stellbewegung des Stößels 39, wodurch die in der Fig. 1 in einer Neutralstellung befindliche Dichtmembran 43 nach unten ausgelenkt wird und die darunter anbringbare Membran 201 der Membranpumpeneinrichtung 2 auf den Ventilsitz 220 gepresst werden kann, um dadurch die gewünschte Blockierwirkung für den Zustromkanal 213 hervorzurufen.

Die Auslass-Stelleinrichtung 14, die rein exemplarisch für eine Beeinflussung des dem Abstromkanal 214 zugeordneten Membranventils 203 vorgesehen ist, umfasst ein zweites Arbeitsfluidventil 82, das über eine Steuerleitung 84 mit der Steuereinrichtung 70 verbunden ist, um eine elektrische Ansteuerung des Arbeitsfluidventils 82 zu ermöglichen. Ferner ist das Arbeitsfluidventil 82 fluidisch mit dem ersten Steuerfluidanschluss 78 verbunden und über eine zweite Arbeitsfluidleitung 86 mit der Auslass-Stelleinrichtung 14 fluidisch kommunizierend gekoppelt. Dementsprechend kann das Arbeitsfluidventil 82, bei dem es sich insbesondere um ein 3/3-Wege-Piezo- Druckregelventil handeln kann, wahlweise eine Versorgung der zweiten Stelleinrichtung 14 mit druckbeaufschlagtem Arbeitsfluid oder eine Entlüftung der zweiten Stelleinrichtung 14 bewirken, wobei die Entlüftung über einen zweiten Entlüftungskanal 88 bis zu einem zweiten Schalldämpfer 90 erfolgt.

Wie aus der Darstellung der Fig. 1 hervorgeht, führt eine Beaufschlagung der Auslass-Stelleinrichtung 14 mit druckbeaufschlagtem Fluid zu einer Stellbewegung des Stößels 39, wodurch die in der Fig. 1 in einer Neutralstellung nach unten ausgelenkte Dichtmembran 43, die die darunter anbringbare Membran 201 der Membranpumpeneinrichtung 2 auf den Ventilsitz 224 presst, wodurch eine Blockierwirkung für den Abstromkanal 214 hervorgerufen wird, in eine Funktionsstellung gebracht werden kann, in der die Dichtmembran 43 zumindest nahezu eben ist, wie dies in der Fig. 1 dargestellt ist und wodurch die darunter anbringbare Membran 201 der Membranpumpeneinrichtung 2 aufgrund ihrer Elastizitätseigenschaften vom Ventilsitz 224 abgehoben wird und den Abstromkanal 214 freigibt.

Für eine Druckregelung in der Steuerkammer 8 ist ein Drucksensor 91 vorgesehen, der über eine Sensorleitung 92 elektrisch mit der Steuereinrichtung 70 verbunden ist und der rein exemplarisch mittels einer Fluidleitung 94 in fluidisch kommunizierender Verbindung mit der ersten Steuerfluidleitung 33 steht. Hierdurch kann der Drucksensor 91 den in der Steuerkammer 8 vorliegenden Fluiddruck ermitteln und als elektrisches Signal über die Sensorleitung 92 an die Steuereinrichtung 70 weiterleiten.

Beispielhaft ist zusätzlich dem zweiten Arbeitsfluidventil 82 ein Drucksensor 93 zugeordnet, der rein exemplarisch in das zweite Arbeitsfluidventil 82 integriert ist und über die zugeordnete zweite Steuerleitung 84 elektrisch mit der Steuereinrichtung 70 verbunden ist.

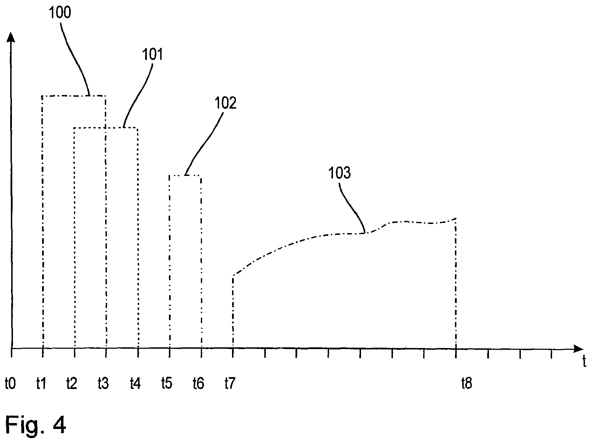

In der Fig. 4 ist ein streng schematischer, modellhafter Ablauf für die Ansteuersignale 101 bis 104 dargestellt, die zur Ansteuerung der einzelnen Komponenten der Betätigungseinrichtung 1 von der Steuereinrichtung 70 bereitgestellt werden. Dabei sind weder die Signalpegel noch die Zeitabschnitte maßstäblich gewählt.

Zu einem Zeitpunkt t0 wird von der Steuereinrichtung 70 kein Ansteuersignal ausgegeben. Somit befinden sich die beiden Stelleinrichtungen 9, 14 jeweils in einer Ruhestellung, in der das zugeordnete Stellglied 38 die jeweilige Dichtmembran 43 jeweils von der Montagefläche 7 abragen lässt und somit eine Deformation der Membran der jeweils gegenüberliegenden Membranventile 202, 203 gewährleistet ist. Dies geht aus der Fig. 3 anhand der strichpunktierten Membran der Membranventile 202, 203 hervor. Somit sind zu diesem Zeitpunkt sowohl der Zustromkanal 213 als auch der Abstromkanal 214 blockiert.

Zu einem Zeitpunkt t1 gibt die Steuereinrichtung 70 ein Ansteuersignal 100 für das erste Arbeitsfluidventil 81 aus, wodurch dieses aus einer Entlüftungsstellung in eine Belüftungsstellung geschaltet wird und eine Druckbeaufschlagung der Einlass-Stelleinrichtung 9 erfolgt. Aufgrund dieser Druckbeaufschlagung bewegt sich der Stößel 39 der Einlass-Stelleinrichtung 9 aus der Neutralstellung gemäß der Fig. 3, in der die Dichtmembran 43 von der Montagefläche 7 abragt und die in Fig. 3 strichtpunktiert dargestellte Membran des zugeordneten Membranventils 202 deformiert wird, in eine Funktionsstellung, wie sie in der Fig. 3 gestrichelt dargestellt ist. Dadurch wird das gegenüberliegend anbringbare Membranventil 202 der Membranpumpeneinrichtung 2 geöffnet, wie dies in der Fig. 3 gestrichelt dargestellt ist, und ermöglicht zumindest prinzipiell einen Zustrom von Fluid vom Eingangsanschluss 204 in die Membranpumpe 200.

Zu einem Zeitpunkt t2 gibt die Steuerschaltung 70 ein Ansteuersignal 101 an das zweite Steuerfluidventil 75 aus, wodurch dieses aus einer Blockierstellung in eine Freigabestellung geschaltet wird und eine Unterdruckbeaufschlagung der Steuerkammer 8 erfolgt. Durch diese Unterdruckbeaufschlagung wird die Membran 201 der Membranpumpe 200 in die Steuerkammer 8 gesaugt und legt sich an der Anlagefläche 31 des Formkörpers 26 an, so dass sie die konvex gewölbte Ansaugstellung 181 einnimmt. Zu diesem Zeitpunkt weist die Pumpkammer 227 der Membranpumpe 200 ein maximales Volumen auf, wobei im Zuge der Deformation der Membran 201 das zu fördernde Fluid vom Eingangsanschluss 204 in die Pumpkammer 227 gesaugt wird.

Zu einem Zeitpunkt t3 schaltet die Steuerschaltung 70 das Ansteuersignal 100 für das erste Arbeitsfluidventil 81 ab, wodurch dieses aus der Belüftungsstellung in die Entlüftungsstellung geschaltet wird und eine Druckbeaufschlagung der Einlass-Stelleinrichtung 9 beendet wird. Dadurch bewegt sich der Stößel 39 der Einlass-Stelleinrichtung 9, insbesondere aufgrund einer Federwirkung einer nicht dargestellten Rückstellfeder, aus der in Fig. 1 dargestellten Funktionsstellung in eine von der Montagefläche 7 abragende Neutralstellung, wie sie in der Fig. 3 strichpunktiert dargestellt ist, so dass das gegenüberliegend anbringbare Membranventil 202 der Membranpumpeneinrichtung 2 geschlossen wird. Zu diesem Zeitpunkt t3 ist der Zustromkanal 213 in der Membranpumpeneinrichtung 2 blockiert und es wird ein Entweichen von Fluid aus der Pumpkammer 227 der Membranpumpe 200 verhindert.

Zu einem Zeitpunkt t4 wird das Ansteuersignal 101 durch die Steuerschaltung 70 abgeschaltet, so dass das zweite Steuerfluidventil 76 wieder die Blockierstellung einnimmt und die Steuerkammer 8 ab diesem Zeitpunkt t4 zunächst einen konstanten Unterdruck aufweist.

Zu einem Zeitpunkt t5 gibt die Steuerschaltung 70 ein Ansteuersignal 102 an das erste Steuerfluidventil 74 aus, wodurch dieses aus einer Blockierstellung in eine Freigabestellung geschaltet wird und eine Druckbeaufschlagung der Steuerkammer 8 erfolgt. Durch diese Druckbeaufschlagung wird das in der Membranpumpe 200 aufgenommene Fluid zusätzlich unter Druck gesetzt. Vorzugsweise ist der Druck in der Steuerkammer 8 so bemessen, dass bei einem nachfolgenden Austragvorgang eine vollständige Verformung der Membran 201 der Membranpumpe 200 aus der konvex gewölbten Ansaugstellung 181 in die konkav gewölbte Austragstellung erfolgt, so dass mit Hilfe der Membranpumpe 200 eine maximale Fluidmenge gefördert werden kann.

Zu einem Zeitpunkt t6 wird das Ansteuersignal 102 durch die Steuerschaltung 70 abgeschaltet, so dass das erste Steuerfluidventil 74 wieder die Blockierstellung einnimmt.

Zu einem Zeitpunkt t7 stellt die Steuerschaltung 70 ein Ansteuersignal 103 für das zweite Arbeitsfluidventil 82 bereit, damit dieses eine geregelte Stellbewegung des der Auslass-Stelleinrichtung 14 zugehörigen Stößels 39 durchführen kann.
Dabei wird das Ansteuersignal 103 in Abhängigkeit von Drucksignalen des Drucksensors 91 in der Steuerschaltung 70 unter Zuhilfenahme eines Beobachteralgorithmus berechnet, um beispielsweise einen möglichst konstanten Fluidmassenstrom aus der Membranpumpe 200 zum Ausgangsanschluss 205 zu bewirken.

Dementsprechend verändert sich die Stellung des Stößels 39 dynamisch während des Fluidfördervorgangs, wobei das Membranventil 203 in der Art eines Proportionalventils betrieben wird. Aufgrund des Überdrucks in der Steuerkammer 8 wird das Fluid in der Pumpkammer 227 der Membranpumpe 200 zum Ausgangsanschluss 205 gedrängt, dieser Vorgang halt vorzugsweise solange an, bis die Membran 201 flächig an der Stirnseite 212 des Stützvorsprungs 211 anliegt und somit die Pumpkammer 227 zumindest nahezu vollständig entleert ist.

Zu einem Zeitpunkt t8 schaltet die Steuerschaltung 70 das Ansteuersignal 103 ab, so dass zu diesem Zeitpunkt der Fördervorgang für die Membranpumpe 200 beendet ist und ein neuer Fördervorgang beginnen kann.

## Patentansprüche

1. Betätigungseinrichtung zur mechanischen Ansteuerung einer Membranpumpeneinrichtung (2) zur Förderung von Fluiden, insbesondere medizinischen Flüssigkeiten für eine Blutbehandlung, mit einem Grundkörper (4), der eine Montagefläche (7) für eine Membranpumpeneinrichtung (2) umfasst und der von einer Steuerfluidleitung (33, 35) durchsetzt ist, die sich von einem Steuerfluidanschluss (78, 79) bis zu einer Steuerfluidmündung (34, 36) erstreckt, die an der Montagefläche (7) ausmündet, sowie mit einer Stelleinrichtung (9, 14), die ein Arbeitsfluidventil (81, 82) umfasst, das für eine Beeinflussung eines Querschnitts einer Arbeitsfluidleitung (85, 86) ausgebildet ist, die den Grundkörper (4) durchsetzt, und wobei das Steuerfluidventil (74, 75) und das Arbeitsfluidventil (81, 82) elektrisch mit einer Steuereinrichtung (70) verbunden sind, die für eine elektrische Ansteuerung des Steuerfluidventils (74, 75) und des Arbeitsfluidventils (81, 20, 82) ausgebildet ist,
wobei in der Steuerfluidleitung (33, 35) ein Steuerfluidventil (74, 75) angeordnet ist, das für eine Beeinflussung eines Querschnitts der Steuerfluidleitung (33, 35) ausgebildet ist, und **dadurch gekennzeichnet, dass**
die Stelleinrichtung (9, 14) einen an der Montagefläche (7) angeordneten Fluidaktor (38) zur Bereitstellung einer Stellbewegung umfasst.

2. Betätigungseinrichtung nach Anspruch 1, **dadurch gekennzeichnet, dass** am Grundkörper (4) ein Steuerfluid-Drucksensor (91) angeordnet ist, der elektrisch mit der Steuereinrichtung (70) verbunden ist und der für eine Erfassung eines Steuerfluiddrucks ausgebildet ist.

3. Betätigungseinrichtung nach Anspruch 2, **dadurch gekennzeichnet, dass** die Steuereinrichtung (70) einen Beobachteralgorithmus umfasst, wobei die Steuereinrichtung (70) derart konfiguriert ist, dass auf der Grundlage des Beobachteralgorithmus ein Fluidmassenstrom einer Membranpumpeneinrichtung (2) in Abhängigkeit von Drucksignalen des Steuerfluid-Drucksensors (91) ermittelt wird.

4. Betätigungseinrichtung nach einem der Ansprüche 1 bis 3, **dadurch gekennzeichnet, dass** der Arbeitsfluidleitung (86) ein Arbeitsfluid-Drucksensor (93) zugeordnet ist, der elektrisch mit der Steuereinrichtung (70) verbunden ist und der für eine Erfassung eines Arbeitsfluiddrucks ausgebildet ist.

5. Betätigungseinrichtung nach einem der Ansprüche 1 bis 4, **dadurch gekennzeichnet, dass** an der Montagefläche (7) eine konkave Steuerkammer (8) als Bewegungsraum für eine Pumpmembran (201) ausgebildet ist und dass die Steuerfluidmündung (34, 36) an einer Oberfläche der Steuerkammer (8) ausmündet.

6. Betätigungseinrichtung nach einem der Ansprüche 1 bis 5, **dadurch gekennzeichnet, dass** im Grundkörper (4) eine Unterdruckleitung (35) ausgebildet ist, die sich von einem Unterdruckanschluss (36) bis zur Steuerkammer (8) oder zur Steuerfluidleitung (33) erstreckt und in der ein Unterdruckventil (75) angeordnet ist, das für eine Beeinflussung eines Querschnitts der Unterdruckleitung (35) ausgebildet ist, wobei das Unterdruckventil (75) elektrisch mit der Steuereinrichtung (70) verbunden ist.

7. Betätigungseinrichtung nach einem der Ansprüche 1 bis 6, **dadurch gekennzeichnet, dass** die Arbeitsfluidleitung (85, 86) fluidisch kommunizierend mit dem Steuerfluidanschluss (78) verbunden ist.

8. Betätigungseinrichtung nach einem der Ansprüche 1 bis 7, **dadurch gekennzeichnet, dass** am Grundkörper (4) eine erste Stelleinrichtung (9) mit einem ersten Arbeitsfluidventil (81) und eine zweite Stelleinrichtung (14) mit einem zweiten Arbeitsfluidventil (82) angeordnet sind, wobei das erste Arbeitsfluidventil (81) als Schaltventil ausgebildet ist und wobei das zweite Arbeitsfluidventil (82) als Proportionalventil, insbesondere als Druckregelventil, ausgebildet ist.

9. Betätigungseinrichtung nach Anspruch 8, **dadurch gekennzeichnet, dass** die Steuereinrichtung derart konfiguriert ist, dass eine Ansteuerung des zweiten Arbeitsfluidventils (82) durch die Steuereinrichtung (70) erfolgt, die einen Fluidmassenstrom in einer Membranpumpeneinrichtung (2) ermittelt und einen Drucksollwert für das zweite Arbeitsfluidventil (82) in Abhängigkeit vom ermittelten Fluidmassenstrom derart bestimmt, dass während einer Austragphase der Membranpumpeneinrichtung (2) ein konstanter Fluidmassenstrom erzeugt wird.

10. Verfahren zum Betreiben einer Betätigungseinrichtung zur mechanischen Ansteuerung einer Membranpumpeneinrichtung (2) nach einem der vorhergehenden Ansprüche, **dadurch gekennzeichnet, dass** eine Ansteuerung des Arbeitsfluidventils (81, 82) durch die Steuereinrichtung (70) erfolgt, die auf der Grundlage eines Beobachteralgorithmus einen Fluidmassenstrom in einer Membranpumpeneinrichtung (2) in Abhängigkeit von Drucksignalen eines Steuerfluid-Drucksensors (91) ermittelt und einen Drucksollwert für das Arbeitsfluidventil (81, 82) in Abhängigkeit vom ermittelten Fluidmassenstrom bestimmt, um während einer Austragphase der Membranpumpeneinrichtung (2) einen konstanten Fluidmassenstrom zu gewährleisten.

11. Verfahren nach Anspruch 10, **dadurch gekennzeichnet, dass** die Steuereinrichtung (70) das Steuerfluidventil (74) und das Unterdruckventil (75) während einer Austragphase der Membranpumpeneinrichtung (2) derart ansteuert, dass die Steuerfluidleitung (34) und die Unterdruckleitung (36) gesperrt sind.

12. Membranpumpe zur Förderung von Fluiden, insbesondere medizinischen Flüssigkeiten für eine Blutbehandlung, mit einer Betätigungseinrichtung (1) nach einem der Ansprüche 1 bis 9 und einer Membranpumpeneinrichtung (2).

13. Membranpumpe nach Anspruch 12, **dadurch gekennzeichnet, dass** die Membranpumpeneinrichtung (2) aufweist:
einen Pumpenkammerkörper (253), in dem eine Ausnehmung ausgebildet ist, die zur Bildung einer Pumpenkammer (252) von einer elastischen Membran (201) verschlossen ist,
einen Zustrompfad (219), der einen Eingangsanschluss (204) mit einer Einlassöffnung (215) der Pumpenkammer (252) verbindet,
einen Abstrompfad (223), der eine Auslassöffnung (216) der Pumpenkammer (252) mit einem Ausgangsanschluss (254) verbindet,
einen in dem Zustrompfad (219) vorgesehenen Einlassventil (202) zur Beeinflussung der Flüssigkeitsströmung in dem Zustrompfad und einen in dem Abstrompfad (223) vorgesehenen Auslassventil (203) zur Beeinflussung der Flüssigkeitsströmung in dem Abstrompfad.

14. Blutbehandlungsvorrichtung, insbesondere Dialysevorrichtung, mit einem Behältnis zur Bereitstellung einer medizinischen Flüssigkeit, insbesondere einer Antikoagulations-Lösung, und einer Membranpumpe nach Anspruch 12 oder 13 zum Fördern einer medizinischen Flüssigkeit, insbesondere einer Antikoagulations-Lösung.

## Claims

1. Operating device for mechanically controlling a membrane pump device (2) for conveying fluids, in particular medical liquids for blood treatment, having a base body (4) which comprises a mounting face (7) for a membrane pump device (2) and through which a control fluid line (33, 35) passes which extends from a control fluid port (78, 79) to a control fluid opening (34, 36) which opens at the mounting face (7), and having an adjustment device (9, 14) which comprises a working fluid valve (81, 82) which is designed to influence a cross section of a working fluid line (85, 86) which passes through the base body (4), and wherein the control fluid valve (74, 75) and the working fluid valve (81, 82) are electrically connected to a control device (70) which is designed to electrically control the control fluid valve (74, 75) and the working fluid valve (81, 20, 82), wherein a control fluid valve (74, 75) is arranged in the control fluid line (33, 35), which control fluid valve is designed to influence a cross section of the control fluid line (33, 35), and **characterized in that** the adjustment device (9, 14) comprises a fluid actuator (38) arranged on the mounting face (7) for providing an adjustment movement.

2. Operating device according to claim 1, **characterised in that** a control fluid pressure sensor (91) is arranged on the base body (4), which control fluid pressure sensor is electrically connected to the control device (70) and is designed to detect a control fluid pressure.

3. Operating device according to claim 2, **characterised in that** the control device (70) includes an observer algorithm, the control device (70) being configured such that, on the basis of the observer algorithm, a mass flow rate of fluid of a membrane pump device (2) is determined depending on pressure signals of the control fluid pressure sensor (91).

4. Operating device according to any of claims 1 to 3, **characterised in that** a working fluid pressure sensor (93) is associated with the working fluid line (86), which working fluid pressure sensor is electrically connected to the control device (70) and is designed to detect a working fluid pressure.

5. Operating device according to any of claims 1 to 4, **characterised in that** a concave control chamber (8) is formed on the mounting face (7) as a movement chamber for a pump membrane (201), and **in that** the control fluid opening (34, 36) opens at a surface of the control chamber (8).

6. Operating device according to any of claims 1 to 5, **characterised in that** a vacuum line (35) is formed in the base body (4), which vacuum line extends from a vacuum port (36) to the control chamber (8) or to the control fluid line (33) and in which a vacuum valve (75) is arranged which is designed to influence a cross section of the vacuum line (35), the vacuum valve (75) being electrically connected to the control device (70).

7. Operating device according to any of claims 1 to 6, **characterised in that** the working fluid line (85, 86) is fluidically connected to the control fluid port (78).

8. Operating device according to any of claims 1 to 7, **characterised in that** a first adjustment device (9) having a first working fluid valve (81) and a second adjustment device (14) having a second working fluid valve (82) are arranged on the base body (4), the first working fluid valve (81) being in the form of a switch valve and the second working fluid valve (82) being in the form of a proportional valve, in particular in the form of a pressure regulating valve.

9. Operating device according to claim 8, **characterised in that** the control device is configured such that the second working fluid valve (82) is controlled by the control device (70), which determines a mass flow rate of fluid in a membrane pump device (2) and determines a target pressure value for the second working fluid valve (82) depending on the determined mass flow rate of fluid in such a manner that a constant mass flow rate of fluid is generated during a discharge phase of the membrane pump device (2).

10. Method for operating an operating device for mechanically controlling a membrane pump device (2) according to any of the preceding claims, **characterised in that** the working fluid valve (81, 82) is controlled by the control device (70) which, on the basis of an observer algorithm, determines a mass flow rate of fluid in a membrane pump device (2) depending on pressure signals of a control fluid pressure sensor (91) and determines a target pressure value for the working fluid valve (81, 82) depending on the determined mass flow rate of fluid in order to ensure a constant mass flow rate of fluid during a discharge phase of the membrane pump device (2).

11. Method according to claim 10, **characterised in that** the control device (70) controls the control fluid valve (74) and the vacuum valve (75) during a discharge phase of the membrane pump device (2) in such a manner that the control fluid line (34) and the vacuum line (36) are blocked.

12. Membrane pump for conveying fluids, in particular medical liquids for blood treatment, having an operating device (1) according to any of claims 1 to 9 and a membrane pump device (2).

13. Membrane pump according to claim 12, **characterised in that** the membrane pump device (2) comprises:
a pump chamber body (253) in which there is formed a recess which is closed by a resilient membrane (201) to form a pump chamber (252),
an inflow path (219) which connects an input port (204) to an inlet opening (215) of the pump chamber (252),
an outflow path (223) which connects an outlet opening (216) of the pump chamber (252) to an output port (254),
an inlet valve (202) provided in the inflow path (219) for influencing the flow of liquid in the inflow path, and an outlet valve (203) provided in the outflow path (223) for influencing the flow of liquid in the outflow path.

14. Blood treatment apparatus, in particular dialysis device, having a container for providing a medical liquid, in particular an anticoagulation solution, and a membrane pump according to either claim 12 or 13 for conveying a medical liquid, in particular an anticoagulation solution.

## Revendications

1. Dispositif d'actionnement pour la commande mécanique d'un dispositif de pompe à membrane (2) pour le refoulement de fluides, en particulier de liquides médicaux pour un traitement du sang, avec un corps de base (4) qui comporte une surface de montage (7) pour un dispositif de pompe à membrane (2) et qui est traversé par une conduite à fluide de commande (33, 35) qui s'étend d'un raccord de fluide de commande (78, 79) jusqu'à une bouche de fluide de commande (34, 36) qui débouche sur la surface de montage (7), ainsi qu'avec un dispositif de réglage (9, 14) qui comporte une vanne à fluide de travail (81, 82) qui est réalisée pour une influence d'une section transversale d'une conduite de fluide de travail (85, 86) qui traverse le corps de base (4), et dans lequel la vanne à fluide de commande (74, 75) et la vanne à fluide de travail (81, 82) sont reliées électriquement à un dispositif de commande (70) qui est réalisé pour une commande électrique de la vanne à fluide de commande (74, 75) et de la vanne à fluide de travail (81, 20, 82), dans lequel une vanne à fluide de commande (74, 75) est agencée dans la conduite à fluide de commande (33, 35), laquelle vanne est réalisée pour une influence d'une section transversale de la conduite à fluide de commande (33, 35), et **caractérisé en ce que** le dispositif de réglage (9, 14) comporte un actionneur à fluide (38) agencé sur la surface de montage (7) pour la fourniture d'un mouvement de réglage.

2. Dispositif d'actionnement selon la revendication 1, **caractérisé en ce qu'**un capteur de pression de fluide de commande (91) est agencé sur le corps de base (4), lequel capteur est relié électriquement au dispositif de commande (70) et est réalisé pour une détection d'une pression de fluide de commande.

3. Dispositif d'actionnement selon la revendication 2, **caractérisé en ce que** le dispositif de commande (70) comporte un algorithme d'observateur, dans lequel le dispositif de commande (70) est configuré de telle manière que sur la base de l'algorithme d'observateur un courant de masse de fluide d'un dispositif de pompe à membrane (2) soit déterminé en fonction de signaux de pression du capteur de pression de fluide de commande (91).

4. Dispositif d'actionnement selon l'une des revendications 1 à 3, **caractérisé en ce qu'**un capteur de pression de fluide de travail (93) est associé à la conduite de fluide de travail (86), lequel est relié électriquement au dispositif de commande (70) et lequel est réalisé pour une détection d'une pression de fluide de travail.

5. Dispositif d'actionnement selon l'une des revendications 1 à 4, **caractérisé en ce qu'**une chambre de commande concave (8) est réalisée sur la surface de montage (7) comme espace de déplacement pour une membrane à pompe (201) et que la bouche de fluide de commande (34, 36) débouche sur une surface de la chambre de commande (8).

6. Dispositif d'actionnement selon l'une des revendications 1 à 5, **caractérisé en ce qu'**une conduite de dépression (35) est réalisée dans le corps de base (4), laquelle s'étend d'un raccord de dépression (36) jusqu'à la chambre de commande (8) ou à la conduite à fluide de commande (33) et dans laquelle une vanne de dépression (75) est agencée, laquelle est réalisée pour une influence d'une section transversale de la conduite de dépression (35), dans lequel la vanne de dépression (75) est reliée électriquement au dispositif de commande (70).

7. Dispositif d'actionnement selon l'une des revendications 1 à 6, **caractérisé en ce que** la conduite de fluide de travail (85, 86) est reliée en communication fluidique au raccord de fluide de commande (78).

8. Dispositif d'actionnement selon l'une des revendications 1 à 7, **caractérisé en ce qu'**un premier dispositif de réglage (9) avec une première vanne à fluide de travail (81) et un second dispositif de réglage (14) avec une seconde vanne à fluide de travail (82) sont agencés sur le corps de base (4), dans lequel la première vanne à fluide de travail (81) est réalisée comme vanne de commutation et dans lequel la seconde vanne à fluide de travail (82) est réalisée comme vanne proportionnelle, en particulier comme vanne de régulation de pression.

9. Dispositif d'actionnement selon la revendication 8, **caractérisé en ce que** le dispositif de commande est configuré de telle manière qu'une commande de la seconde vanne à fluide de travail (82) soit effectuée par le dispositif de commande (70), qui détermine un courant de masse de fluide dans un dispositif de pompe à membrane (2) et détermine une valeur de consigne de pression pour la seconde vanne à fluide de travail (82) en fonction du courant de masse de fluide déterminé de telle manière que pendant une phase d'évacuation du dispositif de pompe à membrane (2) un courant de masse de fluide constant soit généré.

10. Procédé de fonctionnement d'un dispositif d'actionnement pour la commande mécanique d'un dispositif de pompe à membrane (2) selon l'une des revendications précédentes, **caractérisé en ce qu'**une commande de la vanne à fluide de travail (81, 82) est effectuée par le dispositif de commande (70) qui détermine sur la base d'un algorithme d'observateur un courant de masse de fluide dans un dispositif de pompe à membrane (2) en fonction de signaux de pression d'un capteur de pression de fluide de commande (91) et détermine une valeur de consigne de pression pour la vanne à fluide de travail (81, 82) en fonction du courant de masse de fluide déterminé, afin de garantir pendant une phase d'évacuation du dispositif de pompe à membrane (2) un courant de masse de fluide constant.

11. Procédé selon la revendication 10, **caractérisé en ce que** le dispositif de commande (70) commande la vanne à fluide de commande (74) et la vanne de dépression (75) pendant une phase d'évacuation du dispositif de pompe à membrane (2) de telle manière que la conduite à fluide de commande (34) et la conduite de dépression (36) soient bloquées.

12. Pompe à membrane pour le refoulement de fluides, en particulier de liquides médicaux pour un traitement du sang, avec un dispositif d'actionnement (1) selon l'une des revendications 1 à 9, et un dispositif de pompe à membrane (2).

13. Pompe à membrane selon la revendication 12, **caractérisée en ce que** le dispositif de pompe à membrane (2) présente :
un corps de chambre de pompe (253), dans lequel un évidement est réalisé,
lequel est fermé pour la formation d'une chambre de pompe (252) par une membrane élastique (201),
un trajet d'afflux (219) qui relie un raccord d'entrée (204) à une ouverture d'admission (215) de la chambre à pompe (252),
un trajet d'échappement (223) qui relie une ouverture de sortie (216) de la chambre de pompe (252) à un raccord de sortie (254),
une vanne d'entrée (202) prévue dans le trajet d'afflux (219) pour l'influence du courant de liquide dans le trajet d'afflux et une vanne de sortie (203) prévue dans le trajet d'échappement (223) pour l'influence du courant de liquide dans le trajet d'échappement.

14. Dispositif de traitement du sang, en particulier dispositif de dialyse, avec un récipient pour la fourniture d'un liquide médical, en particulier d'une solution anticoagulation, et d'une pompe à membrane selon la revendication 12 ou 13, pour le refoulement d'un liquide médical, en particulier d'une solution anticoagulation.
